# EUROPEAN PATENT SPECIFICATION

(11) **EP 3 100 769 B1**
(45) Date of publication and mention of the grant of the patent: **17.01.2024**
(21) Application number: 16172503.1
(22) Date of filing: 01.06.2016
(51) Int. Cl.: A62B 7/14

(54) **RESPIRATORY MASKS FOR USE IN AIRCRAFTS**
ATEMMASKEN ZUR VERWENDUNG IN FLUGZEUGEN
MASQUE RESPIRATOIRE DESTINÉ À ÊTRE UTILISÉ DANS DES AÉRONEFS

(30) Priority: 02.06.2015 IN 2778CH2015
(43) Date of publication of application: 07.12.2016
(73) Proprietor: Airbus Group India Private Limited, Karnataka, Bangalore (IN)
(72) Inventor: SHARMA, Anurag, Bangalore 560048 Karnataka (IN)
(74) Representative: Ribeiro, James Michael

(56) References cited:
- US-A- 3 675 649
- US-A- 4 648 397
- US-A1- 2012 160 244

## Description

**A** reference is made to an US Application Number 12/748,473 filed on March 29, 2010 and entitled "Adaptable demand dilution oxygen regulator for use in aircrafts" and an US Application Number 13/645,519 filed on October 05, 2012 and entitled "Adaptable demand dilution oxygen regulator for use in aircrafts". A reference is also made to an US Application Number 13/390,517 filed on February 14, 2012 and entitled "Adaptable oxygen regulator system and method with an electronic control device".

### TECHNICAL FIELD

Embodiments of the present subject matter generally relate to respiratory masks, and more particularly, to respiratory masks for use in aircrafts.

### BACKGROUND

Crew members in a cockpit of an aircraft may suffer from several dangerous and catastrophic emergencies. For example, the crew members may suffer from respiratory related emergencies, such as hyperventilation and hypoxia. These emergencies may occur due to smoke in the cockpit, reduction in pressure level due to height of the aircraft, and the like. For example, smoke in the cockpit may occur due to short circuit, equipment failure, insulation breakdown, and the like.

Typically, during such emergencies, the crew member may be provided with a respiratory mask and further, the crew member may have to manually operate a regulator of the respiratory mask to obtain the needed respiratory gases for a smooth respiration. Manual operation of the regulator may not be possible in emergencies, such as smoky environments, due to heavy workload and/or difficulty in locating switches for suppressing the smoke. Also, manual operation of the regulator in such emergencies may need crew member's attention, which may result in distracting the crew member from other needed vital operations. A known respiratory mask for use in an aircraft is disclosed in US 2012/160244 A1.

### SUMMARY

A respiratory mask for use in an aircraft is disclosed. According to one aspect of the present invention, the respiratory mask includes a plurality of sensors for monitoring either cockpit air for parameters capable of affecting oxygen level and/or health of a crew member for parameters capable of causing respiratory disorder. Further, the plurality of sensors provide associated output signals. Furthermore, the respiratory mask includes a regulator electronically coupled to the sensors for automatically switching between operating modes to supply the respiratory gas to the crew member based on the associated output signals of the sensors. The operating modes may include a dilution mode, an emergency mode and a recirculation mode, wherein the respiratory gas comprises a combination of exhaled gas and oxygen during the recirculation mode.

According to another aspect of the present invention, information associated with either cockpit air for parameters capable of affecting oxygen level and/or health of a crew member for parameters capable of causing respiratory disorder is received. Further, switching between operating modes to supply a respiratory gas to the crew member is automatically performed based on the information received. The operating modes may include a dilution mode, an emergency mode and a recirculation mode, wherein the respiratory gas comprises a combination of exhaled gas and oxygen during the recirculation mode.

The respiratory mask and method disclosed herein may be implemented in any means for achieving various aspects. Other features will be apparent from the accompanying drawings and from the detailed description that follow.

### BRIEF DESCRIPTION OF THE DRAWINGS

Various embodiments are described herein with reference to the drawings, wherein:
**FIG. 1** is a block diagram showing major components of an example respiratory mask for use in an aircraft and their connectivity;
**FIG. 2** is a perceptive view of an example respiratory mask for use in an aircraft, such as those shown in Fig. 1;
**FIG.3** is a flow diagram illustrating a method for automatically switching between operating modes of a respiratory mask, in accordance with the present subject matter.

The drawings described herein are for illustration purposes only and are not intended to limit the scope of the present disclosure in any way.

### DETAILED DESCRIPTION

In the following detailed description of the embodiments of the present subject matter, references are made to the accompanying drawings that form a part hereof, and in which are shown by way of illustration specific embodiments in which the present subject matter may be practiced. These embodiments are described in sufficient detail to enable those skilled in the art to practice the present subject matter, and it is to be understood that other embodiments may be utilized and that changes may be made without departing from the scope of the present subject matter. The following detailed description is, therefore, not to be taken in a limiting sense, and the scope of the present subject matter is defined by the appended claims.

Generally, a crew member of an aircraft is provided with a respiratory mask for obtaining needed respiratory gas during emergencies. Such emergencies may include smoke in a cockpit of the aircraft, pressurization loss, air contamination, and the like. However, such respiratory masks may be generally bulky and removal of the respiratory mask from a stowage located beside a seat of the crew member for wearing it may be cumbersome and may need significant time. During this process, the crew member may ends up inhaling the smoke.

Embodiments described herein provide a respiratory mask for use by the crew member in an aircraft. The crew member may include a pilot, flight attendant, flight medic, and the like. In an embodiment, the respiratory mask may include a plurality of sensors for monitoring either cockpit air for parameters capable of affecting oxygen level and/or crew member's health for parameters capable of causing respiratory disorder. The parameters capable of affecting oxygen level may be understood as presence of smoke in a cockpit of the aircraft, pressure level inside the cockpit, and contaminants present in cockpit ambient air. Similarly, the parameters capable of causing respiratory disorder may be breathing rate of a crew member in the aircraft, carbon dioxide level present in exhaled gas of the crew member, partial pressure of oxygen present in crew member's blood, and tissue oxygen saturation of the crew member. Further, the sensors may provide associated output signals. Furthermore, the respiratory mask may include a regulator electronically coupled to the sensors for automatically switching between operating modes to supply the respiratory gas to the crew member based on the associated output signals of the sensors. For example, the operating modes may include a dilution mode, an emergency mode and a recirculation mode. The respiratory gas may include oxygen during the emergency mode, a combination of the exhaled gas including carbon dioxide (CO₂) and gases already present in the mouthpiece and regulator cavity during the recirculation mode, and a combination of air inside the cockpit or air mixed with oxygen during the dilution mode.

Thus, the respiratory mask functions continuously as a respiratory aid during long haul crew member operations to prevent sudden incapacitation due to the emergencies and preserves bottled oxygen supply in the aircraft. Also, the respiratory mask is lightweight. Therefore, the mask can be worn by the crew member comfortably throughout the flight.

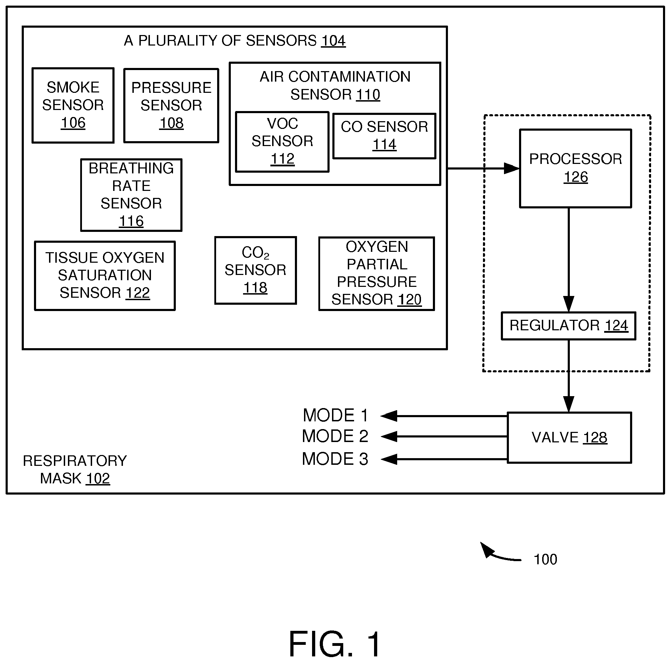

The respiratory mask 102, in accordance with an example of the present subject matter, is explained in detail with reference to FIG. 1 to 3, where the reference numerals indicate key components of the respiratory mask 102.

**FIG. 1** is a block diagram 100 of a respiratory mask 102 for use in an aircraft (not shown in the figures), according to one embodiment. The respiratory mask 102 may be worn by a crew member comfortably throughout the flight of the aircraft. For example, the crew member may include a pilot, flight attendant, flight medic, and the like. In addition, the respiratory mask 102 may be operable throughout the flight of the aircraft for protecting the crew member from several dangerous and catastrophic emergencies, thereby avoiding hazardous situations such as crash of the aircraft. For example, the emergencies may be respiratory related emergencies, such as hyperventilation, hypoxia and the like.

In accordance with the present subject matter, the respiratory mask 102 may further include a regulator 124 and a plurality of sensors 104. In one implementation, the regulator 124 may be electronically coupled to the plurality of sensors 104 through a processor 126. In one implementation, the regulator 124 and the processor 126 may be separate components coupled to each other. In another implementation, the regulator 124 may include the processor 126. Further, the plurality of sensors 104 may include smoke sensor 106, pressure sensor 108, air contamination sensor 110, breathing rate sensor 116, tissue oxygen saturation sensor 122, carbon dioxide (CO₂) sensor 118, and oxygen partial pressure sensor 120. In one example, the air contamination sensor 110 may include Volatile Organic Compound (VOC) sensor 112 and Carbon monoxide (CO) sensor 114. The processor 126 receives output of the plurality of sensors 104 as an input. The output of the plurality of sensors 104 is then processed by the processor 126 and sent to the regulator 124. The regulator 124, upon receiving the processed output, actuates one or more valves 128 for switching either to mode 1 or mode 2 or mode 3 based on the received processed output. In one embodiment, the one or more valves 128 may reside inside the regulator 124. For the purpose of simplicity of explanation, the mode 1 may be understood as an emergency mode, the mode 2 may be understood as a recirculation mode, and the mode 3 may be understood as a dilution mode. Further, actuation of the one or more valves 128 to switch either to mode 1 or mode 2 or mode 3 may help to supply a respiratory gas to the crew member. The respiratory gas may include oxygen which may be supplied during the emergency mode. The respiratory gas may also include the exhaled gas which may be supplied during the recirculation mode. The respiratory gas may further include a combination of air inside the cockpit or the cockpit ambient air progressively mixed with more oxygen as altitude increases may be supplied during the dilution mode.

Further, the regulator 124 may actuate the one or more valves 128 based on the output of the sensors 104. In one example, the regulator 124 may actuate the one or more valves 128 when the output indicates emergency conditions. The emergency conditions may be understood as conditions when there is presence of smoke in the cockpit, the pressure level present inside the cockpit is lower than a predetermined pressure level, the partial pressure of the oxygen present in the crew member's blood is lower than a predetermined partial pressure of the oxygen, the carbon dioxide level present in the exhaled gas is higher than a predetermined carbon dioxide level, and/or the breathing rate of the crew member is deviated from a predetermined breathing rate.

The predetermined pressure level may be understood as a level of the pressure inside the cockpit at which the crew member can breathe comfortably without any hurdle. For example, the predetermined pressure level may be considered as a level of the pressure at a height of 6000 feet from the sea level. Similarly, the predetermined partial pressure of the oxygen may be understood as a level of the partial pressure of the oxygen at which mental acuity of the crew member may start to be affected. For example, the predetermined partial pressure of the oxygen may be in between 94% to 95%. In a like manner, for the purpose of simplicity of the explanation, the predetermined carbon dioxide level may be understood as a level of the carbon dioxide present in the exhaled gas from the body of the crew member such that the respiratory system of the crew member is healthy. Likewise, the predetermined breathing rate may be understood as breathing rate at which the crew member breathes comfortably. The breathing rate may be understood as, for example, number of breaths per minute. Alternatively, the breathing rate may be understood, for example, as an expiration volume in liter/sec or an expiration volume per breath. The mode of the respiratory mask 102 in accordance with this implementation may be understood as emergency mode. Such above explained emergency conditions may result in lack of oxygen and the crew member may get respiratory disorder, such as hypoxia, due to lack of oxygen which may lead to unconsciousness of the crew member.

To overcome the respiratory disorder due to the lack of oxygen, the crew member may be forced to inhale oxygen so that the level of oxygen in the blood can be retained to a level where the crew member can breathe smoothly. Therefore, the regulator 124 enables providing oxygen by actuating the one or more valves 128. Thus, inhalation of oxygen may protect the crew member from hypoxia caused by the above explained emergency conditions. Protection of the crew member using oxygen is not limited only to hypoxia and inhalation of oxygen may also protect the crew member from the respiratory disorders other than hypoxia.

In another example, the regulator 124 may actuate the one or more valves 128 when the output indicates that a percentage of oxygen present in the blood of the crew member is at least 94%. The regulator 124 may also actuate the one or more valves 128 when the carbon dioxide level present in the exhaled gas is lower than the predetermined carbon dioxide level and/or when the breathing rate of the crew member is higher than the predetermined breathing rate and/or expiration volume is low which may occur during a high stress condition. In such above explained conditions, the crew member may be hyperventilating and may subsequently lead to unconsciousness of the crew member. The mode of the respiratory mask 102 in accordance with this implementation may be understood as recirculation mode. Therefore, it is essential to protect the crew members from these conditions. To overcome the respiratory disorder caused by these conditions, the crew member may be momentarily forced to inhale the exhaled gas till the breathing rate can be controlled. The oxygen may be stored in an oxygen storage kept onboard for supplying when needed. Further, inhalation of the recirculated gas helps the brain to auto-regulate the breathing rate, thereby protecting the crew member from hyperventilation caused by the above explained conditions.

In yet another example, the regulator 124 may actuate the one or more valves 128 to the dilution mode when the output of the sensors 104 indicates any one of the conditions, such as presence of no smoke in the cockpit, the pressure level inside the cockpit is higher than a predetermined pressure level, the partial pressure of the oxygen present in the crew member's blood is higher than a predetermined partial pressure of the oxygen and/or the breathing rate of the crew member is equal to a predetermined breathing rate. The predetermined breathing rate, the predetermined carbon dioxide level, and the predetermined partial pressure of the oxygen may be understood as explained above. The dilution mode may be understood as a mode where the respiratory system of the crew member functions normally, i.e., in which there is no symptoms of either hyperventilation or hypoxia or any other respiratory disorder in the crew member.

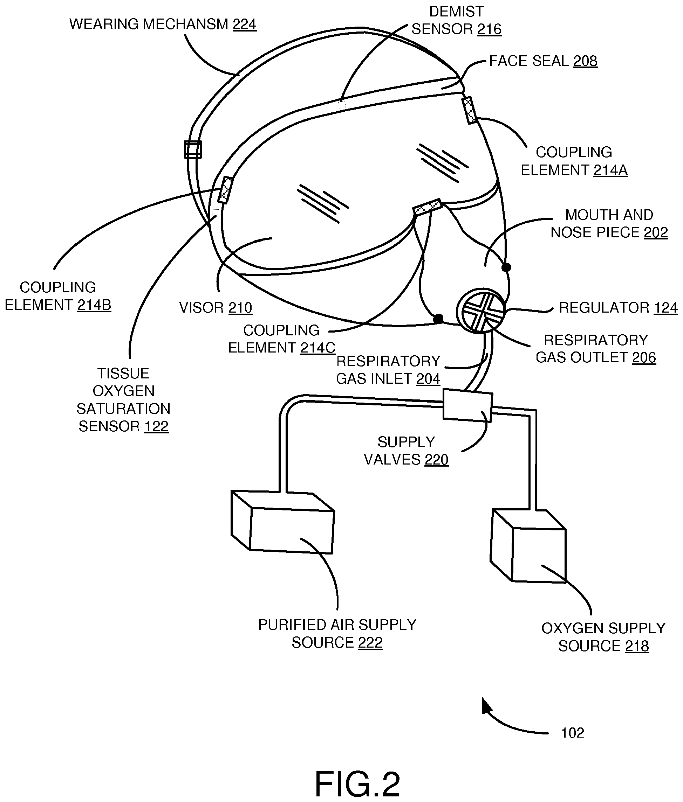

Referring now to FIG. 2 which illustrates a perspective view of a respiratory mask 102 for use in an aircraft, in accordance with an example of the present subject matter. As illustrated in FIG. 2, the respiratory mask 102 may include a mouth and nose piece 202 connected to a regulator 124 similar to as illustrated in FIG. 1. The mouth and nose piece 202 may have smoke sensors (similar to as illustrated in FIG. 1) for sensing presence of smoke in a cockpit of the aircraft and may also have pressure sensors (similar to as illustrated in FIG. 1) for sensing the pressure level present inside the cockpit. The smoke sensors and the pressure sensors may be mounted, for example, around the mouth and nose piece 202. For example, the smoke sensors may be mounted on the regulator 124. The smoke sensors are explained to be mounted on the regulator 124 for the purpose of explanation and can be mounted anywhere on the respiratory mask 102 or can be mounted anywhere in the cockpit for sensing the smoke in the cockpit. Further, the respiratory mask 102 may include a respiratory gas inlet 204 and a respiratory gas outlet 206. The respiratory gas inlet 204 and a respiratory gas outlet 206 may be connected to the mouth and nose piece 202 for the purpose of inhaling the respiratory gas and exhaling the exhaled gas respectively. The respiratory gas inlet 204 may include breathing rate sensors (similar to as illustrated in FIG. 1) disposed therein for sensing a breathing rate of a crew member in the aircraft. Similarly, the respiratory gas outlet 206 may include carbon dioxide sensors (similar to as illustrated in FIG. 1) disposed therein for sensing a carbon dioxide level in an exhaled gas by the crew member. Further, the respiratory mask 102 may include a microphone (not shown in the figures) for the communication purpose. For example, the microphone may be attached to the mouth and nose piece 202. In another example, the microphone may be inbuilt with the mouth and nose piece 202.

Further, the respiratory mask 102 as illustrated in FIG. 2 may include a peripheral face seal 208. The peripheral face seal 208 may be fitted to a visor 210. The visor 210 is removably attached to the respiratory mask 102 with the help of coupling elements 214A, 214B, and 214C. Further, the peripheral face seal 208 enables to airtight the face of the crew member for protecting the face from contaminates present in air of cockpit and heat generated by fumes or smoke. The peripheral face seal 208 may be made of, for example, pliable /compliant materials. Furthermore, the peripheral face seal 210 may include a demist sensor 216, disposed thereon, for sensing the mist on the visor 210. The demist sensor 216 may be communicatively coupled to the regulator 124 for providing a feedback to the regulator 124 that there is a mist on the visor 210 and hence demisting is needed. The regulator 124, upon receiving the feedback, may perform demisting to clear the visor 210 by opening its outflow outlet to reduce outlet pressure and increasing ventilation flow.

Further, the respiratory mask 102 may be connected to an oxygen supply source 218 through one or more supply valves 220. The oxygen supply source 218 may be utilized to deliver oxygen to the crew member when there is demand for oxygen, such as during emergency mode and recirculation mode. For delivering oxygen to the crew member, the regulator 124 may actuate the one or more valves 128 to enable flow of oxygen from the oxygen supply source 218 to the crew member through the respiratory gas inlet 204 and the mouth and nose piece 202. During emergency mode, the respiratory gas may include 100% oxygen supplied from the oxygen supply source 218. Whereas, during recirculation mode, the respiratory gas may include a combination of oxygen from the oxygen supply source 218 and exhaled gas by the crew member.

Furthermore, in accordance with an embodiment of the present subject matter, the respiratory mask 102 may be connected to a purified air supply source 222 through the one or more supply valves 220. The purified air supply source 222 may be utilized to supply purified air to the crew member when air inside the cockpit is contaminated by contaminants, such as Volatile Organic Compounds (VOC) and Carbon Monoxide (CO). The purified air supply source 222 may purify the air inside the cockpit to eliminate contaminants from the air and generate a purified air. The purified air is then supplied to the crew member by the purified air supply source 222 through the respiratory gas inlet 204 and the mouth and nose piece 202. In one implementation, the purified air is supplied during the dilution mode which preserves consumption of oxygen from the oxygen storage. In accordance with another embodiment of the present subject matter, the respiratory mask 102 may include a filter unit (not shown the figures) in the mouth and nose piece 202 to filter the air inside the cockpit and provide purified/filtered air to the crew member. In one embodiment, the respiratory mask 102 may also include a wearing mechanism 224 for facilitating the crew member to wear the respiratory mask 102. The wearing mechanism 224, for example, may be straps connected to each other in such a manner that they can be utilized for wearing the respiratory mask 102. In one example, the wearing mechanism 224 may be an adjustable strap or band which can be fitted around the head of the crew member to wear the respiratory mask 102.

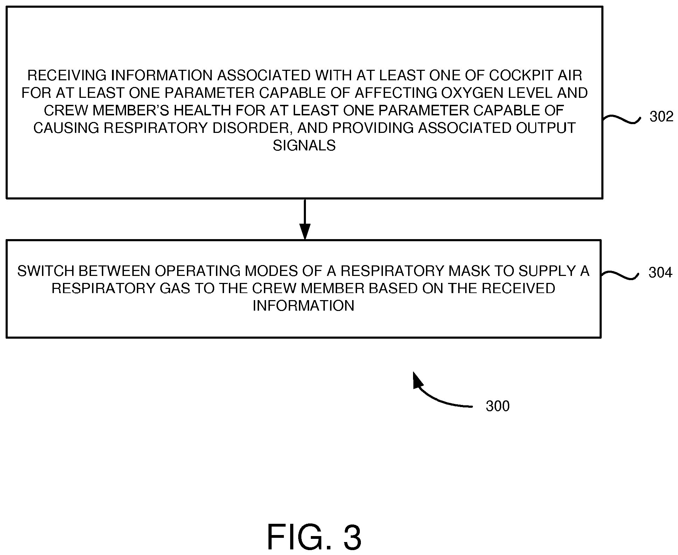

Referring now to FIG. 3 which is a flow diagram 300 illustrating an example method, in accordance with the present subject matter. At step 302, information associated with either cockpit air for parameters capable of affecting oxygen level and/or crew member's health for parameters capable of causing respiratory disorder is received. The parameters capable of affecting oxygen level may be presence of smoke in a cockpit of the aircraft, pressure level inside the cockpit, and contaminants present in cockpit ambient air. Similarly, the parameters capable of causing respiratory disorder may be breathing rate of a crew member in the aircraft, carbon dioxide level present in exhaled gas of the crew member, partial pressure of oxygen present in crew member's blood, and tissue oxygen saturation of the crew member.

At step 304, automatic switching between various operating modes is performed based on the received information. The operating modes may include a dilution mode, an emergency mode, and a recirculation mode. The dilution mode, the emergency mode, and the recirculation mode may be understood as explained above.

The respiratory mask 102 in accordance with the present subject matter may include the visor 210 either covering full face of the crew member or only the eyes of the crew member. The respiratory mask 102 may start functioning when it is removed from a stowage located beside a seat of the crew member. Further, respiratory mask 102 may be wearable during entire flight time due to its light weight. Furthermore, the respiratory mask 102 may be capable to operate in either emergency mode or recirculation mode or dilution mode which reduces consumption of oxygen from the oxygen storage and hence enables the crew member to wear the respiratory mask 102 during entire flight time without any interruption in the supply of the respiratory gas.

It may be noted that the above-described examples of the present solution is for the purpose of illustration only.

The terms "include," "have," and variations thereof, as used herein, have the same meaning as the term "comprise" or appropriate variation thereof. Furthermore, the term "based on", as used herein, means "based at least in part on."

The present description has been shown and described with reference to the foregoing examples.

## Claims

1. A respiratory mask (102) for use in an aircraft, comprising:
a plurality of sensors (104) monitoring at least one of cockpit ambient air for at least one parameter capable of affecting oxygen level and health of a crew member for at least one parameter capable of causing respiratory disorder, and providing associated output signals; and
a regulator (124) electronically coupled to the plurality of sensors (104), wherein the regulator (124) automatically switches between operating modes to supply respiratory gas to the crew member based on the associated output signals of the plurality of sensors (104), wherein the operating modes comprise a dilution mode, an emergency mode, and a recirculation mode, wherein the respiratory gas comprises a combination of exhaled gas and oxygen during the recirculation mode.

2. The respiratory mask (102) of claim 1, wherein the at least one parameter capable of affecting oxygen level is selected from the group consisting of presence of smoke in a cockpit of the aircraft, pressure level inside the cockpit, and contaminants present in the cockpit ambient air.

3. The respiratory mask (102) of claim 1, wherein the at least one parameter capable of causing respiratory disorder is selected from the group consisting of breathing rate of the crew member in the aircraft, carbon dioxide level present in exhaled gas of the crew member, partial pressure of oxygen present in blood of the crew member, and tissue oxygen saturation of the crew member.

4. The respiratory mask (102) of claim 1, wherein the regulator (124) is configured to:
automatically switch to the emergency mode when at least one emergency condition occurs, wherein the at least one emergency condition is selected from the group consisting of presence of smoke in a cockpit, pressure level inside the cockpit is lower than a predetermined pressure level, partial pressure of oxygen present in blood of the crew member is lower than a predetermined partial pressure of oxygen, carbon dioxide level present in exhaled gas is higher than a predetermined carbon dioxide level, breathing rate of the crew member is deviated from a predetermined breathing rate, and tissue oxygen saturation of the crew member is lower than a predetermined tissue oxygen saturation.

5. The respiratory mask (102) of claim 1, wherein the regulator (124) is configured to:
automatically switch to the recirculation mode when a percentage of oxygen in blood of the crew member is at least 94% and one of carbon dioxide level present in exhaled gas is lower than a predetermined carbon dioxide level and breathing rate of the crew member is higher than a predetermined breathing rate.

6. The respiratory mask (102) of claim 1, wherein the regulator (124) is configured to:
automatically switch to the dilution mode when at least one condition occurs, wherein the at least one condition comprises no smoke present in a cockpit, pressure level inside the cockpit is higher than a predetermined pressure level, breathing rate of the crew member is equal to a predetermined breathing rate, carbon dioxide level present in exhaled gas is equal to a predetermined carbon dioxide level, and partial pressure of oxygen present in blood of the crew member is equal to a predetermined partial pressure of oxygen.

7. The respiratory mask (102) of claim 1, wherein the respiratory gas comprises one of oxygen during the emergency mode, and the cockpit ambient air during the dilution mode.

8. The respiratory mask (102) of claim 1, further comprising:
a mouth and nose piece (202) connected to the regulator (124);
a respiratory gas inlet (204) and a respiratory gas outlet (206) connected to the mouth and nose piece (202), wherein at least one breathing rate sensor (116) from the plurality of sensors (104) is disposed in the respiratory gas inlet (204) for sensing breathing rate of the crew member and wherein at least one carbon dioxide sensor (114) from the plurality of sensors (104) is disposed in at least one of the respiratory gas outlet (206) and the mouth and nose piece (202) for sensing carbon dioxide level present in exhaled gas.

9. The respiratory mask (102) of claim 1, further comprising:
a peripheral face seal (208), wherein at least one sensor (120) from the plurality of sensors (104) is disposed around the peripheral face seal (208) for sensing partial pressure of oxygen present in blood of the crew member.

10. A method of using the respiratory mask of claim 1, comprising:
receiving, from a plurality of sensors (104), information associated with at least one of cockpit ambient air for at least one parameter capable of affecting oxygen level and health of a crew member for at least one parameter capable of causing respiratory disorder, and providing associated output signals; and
automatically switching between operating modes of a respiratory mask (102) to supply a respiratory gas to the crew member based on the received information, wherein the operating modes comprise a dilution mode, an emergency mode, and a recirculation mode, wherein the respiratory gas comprises a combination of exhaled gas and oxygen during the recirculation mode.

11. The method of claim 10, wherein the at least one parameter capable of affecting oxygen level is selected from the group consisting of presence of smoke in a cockpit, pressure level inside the cockpit, and contaminants present in the cockpit ambient air.

12. The method of claim 10, wherein the at least one parameter capable of causing respiratory disorder is selected from the group consisting of breathing rate of the crew member in an aircraft, carbon dioxide level present in exhaled gas of the crew member, partial pressure of oxygen present in blood of the crew member, and tissue oxygen saturation of the crew member.

13. The method of claim 10, wherein the respiratory gas comprises one of oxygen during the emergency mode, and at least one of a combination of the cockpit ambient air and the cockpit ambient air mixed with oxygen during the dilution mode.

14. The method of claim 10, wherein the automatically switching between the operating modes to supply the respiratory gas to the crew member based on the received information, comprises:
automatically switching to the emergency mode when at least one emergency condition occurs, wherein the at least one emergency condition comprises presence of smoke in a cockpit, pressure level inside the cockpit is higher than a predetermined pressure level, partial pressure of oxygen present in blood of the crew member is lower than a predetermined partial pressure of the oxygen, carbon dioxide level present in exhaled gas is higher than a predetermined carbon dioxide level, breathing rate of the crew member is deviated from a predetermined breathing rate, and tissue oxygen saturation of the crew member is lower than a predetermined tissue oxygen saturation.

## Patentansprüche

1. Atemmaske (102) zur Verwendung in einem Flugzeug, umfassend:
eine Vielzahl von Sensoren (104), die mindestens eines von der Cockpit-Umgebungsluft auf mindestens einen Parameter, der in der Lage ist, den Sauerstoffgehalt zu beeinflussen, und der Gesundheit eines Besatzungsmitglieds auf mindestens einen Parameter, der in der Lage ist, Atemwegserkrankungen zu verursachen, überwachen und zugehörige Ausgangssignale bereitstellen, und
einen Regler (124), der elektronisch mit den mehreren Sensoren (104) gekoppelt ist, wobei der Regler (124) automatisch zwischen Betriebsmodi wechselt, um Atemgas dem Besatzungsmitglied basierend auf den zugehörigen Ausgangssignalen der mehreren Sensoren (104) zuzuführen, wobei die Betriebsmodi einen Verdünnungsmodus, einen Notfallmodus und einen Rezirkulationsmodus umfassen, wobei das Atemgas eine Kombination aus ausgeatmetem Gas und Sauerstoff während des Rezirkulationsmodus umfasst.

2. Atemmaske (102) nach Anspruch 1, wobei der mindestens eine Parameter, der den Sauerstoffgehalt beeinflussen kann, aus der Gruppe ausgewählt ist, bestehend aus dem Vorhandensein von Rauch in einem Cockpit des Flugzeugs, dem Druckniveau im Cockpit und Verunreinigungen, die in der Cockpit-Umgebungsluft vorhandenen sind.

3. Atemmaske (102) nach Anspruch 1, wobei der mindestens eine Parameter, der eine Atemstörung verursachen kann, aus der Gruppe ausgewählt ist, bestehend aus der Atemfrequenz des Besatzungsmitglieds im Flugzeug, dem Kohlendioxidgehalt im ausgeatmeten Gas des Besatzungsmitglieds, dem Sauerstoffpartialdruck im Blut des Besatzungsmitglieds und der Gewebe-Sauerstoffsättigung des Besatzungsmitglieds.

4. Atemmaske (102) nach Anspruch 1, wobei der Regler (124) dazu beschaffen ist:
automatisch in den Notfallmodus zu wechseln, wenn mindestens eine Notfallbedingung auftritt, wobei die mindestens eine Notfallbedingung aus der Gruppe ausgewählt ist, bestehend aus dem Vorhandensein von Rauch in einem Cockpit, einem Druckniveau im Cockpit, das niedriger ist als ein vorgegebenes Druckniveau, einem Partialdruck von Sauerstoff, der im Blut des Besatzungsmitglieds vorhanden ist und niedriger ist als ein vorgegebener Sauerstoffpartialdruck, der Kohlendioxidgehalt im ausgeatmeten Gas, der höher ist als ein vorgegebener Kohlendioxidgehalt, die Atemfrequenz des Besatzungsmitglieds, die von einer vorgegebenen Atemfrequenz abweicht, und die Gewebe-Sauerstoffsättigung des Besatzungsmitglieds, die niedriger ist als eine vorgegebene Gewebe-Sauerstoffsättigung.

5. Atemmaske (102) nach Anspruch 1, wobei der Regler (124) dazu beschaffen ist:
automatisch in den Rezirkulationsmodus zu wechseln, wenn der Sauerstoffanteil im Blut des Besatzungsmitglieds mindestens 94 % beträgt und eines von dem Kohlendioxidgehalt im ausgeatmeten Gas niedriger als ein vorgegebener Kohlendioxidgehalt und die Atemfrequenz des Besatzungsmitglieds höher als eine vorgegebene Atemfrequenz ist.

6. Atemmaske (102) nach Anspruch 1, wobei der Regler (124) dazu beschaffen ist:
automatisch in den Verdünnungsmodus zu wechseln, wenn mindestens eine Bedingung auftritt, wobei die mindestens eine Bedingung umfasst, dass kein Rauch in einem Cockpit vorhanden ist, der Druckpegel im Cockpit höher ist als ein vorgegebener Druckpegel, die Atemfrequenz des Besatzungsmitglieds gleich einer vorgegebenen Atemfrequenz ist, der Kohlendioxidgehalt im ausgeatmeten Gas gleich einem vorgegebenen Kohlendioxidgehalt ist und der Sauerstoffpartialdruck im Blut des Besatzungsmitglieds gleich einem vorgegebenen Sauerstoffpartialdruck.

7. Atemmaske (102) nach Anspruch 1, wobei das Atemgas im Notfallmodus Sauerstoff und im Verdünnungsmodus die Cockpit-Umgebungsluft umfasst.

8. Atemmaske (102) nach Anspruch 1, ferner umfassend:
ein Mund- und Nasenstück (202), das mit dem Regler (124) verbunden ist,
einen Atemgaseinlass (204) und einen Atemgasauslass (206), die mit dem Mund-und Nasenstück (202) verbunden sind, wobei mindestens ein Atemfrequenzsensor (116) aus der Vielzahl von Sensoren (104) im Atemgaseinlass (204) zum Erfassen der Atemfrequenz des Besatzungsmitglieds angeordnet ist und wobei mindestens ein Kohlendioxidsensor (114) aus der Vielzahl von Sensoren (104) im Atemgasauslass (206) und/oder im Mund- und Nasenstück (202) zum Erfassen des Kohlendioxidgehalts im ausgeatmeten Gas angeordnet ist.

9. Atemmaske (102) nach Anspruch 1, ferner umfassend:
eine periphere Gesichtsdichtung (208), wobei mindestens ein Sensor (120) aus der Vielzahl von Sensoren (104) um die periphere Gesichtsdichtung (208) herum zum Erfassen des Sauerstoffpartialdrucks im Blut des Besatzungsmitglieds angeordnet ist.

10. Verfahren zur Verwendung der Atemmaske nach Anspruch 1, umfassend:
Empfangen, von einer Vielzahl von Sensoren (104), von Informationen, die in Verbindung stehen mit mindestens einem von der Cockpit-Umgebungsluft auf mindestens einen Parameter, der in der Lage ist, den Sauerstoffgehalt zu beeinflussen, und der Gesundheit eines Besatzungsmitglieds auf mindestens einen Parameter, der in der Lage ist, Atemwegserkrankungen zu verursachen, und Bereitstellen zugehöriger Ausgangssignale, und
automatisches Wechseln zwischen Betriebsmodi einer Atemmaske (102), um ein Atemgas dem Besatzungsmitglied basierend auf den empfangenen Informationen zuzuführen, wobei die Betriebsmodi einen Verdünnungsmodus, einen Notfallmodus und einen Rezirkulationsmodus umfassen, wobei das Atemgas eine Kombination aus ausgeatmetem Gas und Sauerstoff während des Rezirkulationsmodus umfasst.

11. Verfahren nach Anspruch 10, wobei der mindestens eine Parameter, der den Sauerstoffgehalt beeinflussen kann, aus der Gruppe ausgewählt ist, bestehend aus dem Vorhandensein von Rauch in einem Cockpit, dem Druckniveau im Cockpit und Verunreinigungen, die in der Cockpit-Umgebungsluft des vorhandenen sind.

12. Verfahren nach Anspruch 10, wobei der mindestens eine Parameter, der eine Atemstörung verursachen kann, aus der Gruppe ausgewählt ist, bestehend aus der Atemfrequenz des Besatzungsmitglieds in einem Flugzeug, dem Kohlendioxidgehalt im ausgeatmeten Gas des Besatzungsmitglieds, dem Sauerstoffpartialdruck im Blut des Besatzungsmitglieds und der Gewebe-Sauerstoffsättigung des Besatzungsmitglieds.

13. Verfahren nach Anspruch 10, wobei das Atemgas eines von Sauerstoff während des Notfallmodus und mindestens eines einer Kombination aus der Cockpit-Umgebungsluft und der Cockpit-Umgebungsluft gemischt mit Sauerstoff während des Verdünnungsmodus umfasst.

14. Verfahren nach Anspruch 10, wobei das automatische Wechseln zwischen den Betriebsmodi, um das Atemgas dem Besatzungsmitglied basierend auf den empfangenen Informationen zuzuführen, umfasst:
automatisches Wechseln in den Notfallmodus, wenn mindestens eine Notfallbedingung auftritt, wobei die mindestens eine Notfallbedingung das Vorhandensein von Rauch in einem Cockpit, ein Druckniveau im Cockpit, das höher ist als ein vorgegebenes Druckniveau, einen Partialdruck von Sauerstoff, der im Blut des Besatzungsmitglieds vorhanden ist und niedriger ist als ein vorgegebener Sauerstoffpartialdruck, den Kohlendioxidgehalt im ausgeatmeten Gas, der höher ist als ein vorgegebener Kohlendioxidgehalt, die Atemfrequenz des Besatzungsmitglieds, die von einer vorgegebenen Atemfrequenz abweicht, und die Gewebe-Sauerstoffsättigung des Besatzungsmitglieds, die niedriger ist als eine vorgegebene Gewebe-Sauerstoffsättigung.

## Revendications

1. Un masque respiratoire (102) destiné à être utilisé dans un aéronef, comprenant :
une pluralité de capteurs (104) contrôlant au moins un airr ambiant du cockpit pour au moins un paramètre capable d'affecter le niveau d'oxygène et la santé d'un membre d'équipage pour au moins un paramètre capable de causer des troubles respiratoires, et fournissant des signaux de sortie associés ; et
un régulateur (124) couplé électroniquement à la pluralité de capteurs (104), dans lequel le régulateur (124) passe automatiquement entre des modes opératoires pour fournir du gaz respiratoire au membre d'équipage en fonction des signaux de sortie associés de la pluralité de capteurs (104), dans lequel les modes de fonctionnement comprenent un mode de dilution, un mode d'urgence et un mode de recirculation, dans lequel le gaz respiratoire comprend une combinaison de gaz expiré et d'oxygène pendant le mode de recirculation.

2. Le masque respiratoire (102) selon la revendication 1, dans lequel au moins un paramètre susceptible d'affecter le niveau d'oxygène est choisi dans le groupe constitué par : la présence de fumée dans le cockpit de l'avion, le niveau de pression à l'intérieur du cockpit et de contaminants présents dans l'air ambiant du cockpit.

3. Le masque respiratoire (102) selon la revendication 1, dans lequel au moins un paramètre susceptible de provoquer un trouble respiratoire est choisi dans le groupe constitué par : une fréquence respiratoire du membre d'équipage de l'avion, un niveau de dioxyde de carbone présent dans les gaz expirés du membre d'équipage, la pression partielle d'oxygène présente dans le sang du membre d'équipage et la saturation en oxygène des tissus du membre d'équipage.

4. Le masque respiratoire (102) selon la revendication 1, dans lequel le régulateur (124) est configuré pour : passer automatiquement en mode d'urgence lorsqu'au moins une condition d'urgence se produit, dans lequel la au moins une condition d'urgence est sélectionnée dans le groupe consistant en présence de fumée dans un cockpit, le niveau de pression à l'intérieur du cockpit est inférieur à un niveau de pression prédéterminé, la pression partielle d'oxygène présent dans le sang du membre d'équipage est inférieure à une pression partielle d'oxygène prédéterminée, le niveau de dioxyde de carbone présent dans le gaz expiré est supérieur à un niveau de dioxyde de carbone prédéterminé, la fréquence respiratoire du membre d'équipage dévied'une fréquence respiratoire prédéterminée, et la saturation en oxygène des tissus du membre d'équipage est inférieure à une saturation en oxygène des tissus prédéterminée.

5. Le masque respiratoire (102) selon la revendication 1, dans lequel le régulateur (124) est configuré pour : passer automatiquement dans le mode de recirculation lorsqu'un pourcentage d'oxygène dans le sang du membre d'équipage est d'au moins 94 % et que l'un des niveaux de dioxyde de carbone présent dans le gaz expiré est inférieur à un niveau de dioxyde de carbone prédéterminé et la fréquence respiratoire du membre d'équipage est supérieure à une fréquence respiratoire prédéterminée.

6. Un masque respiratoire (102) selon la revendication 1, dans lequel le régulateur (124) est configuré pour : passer automatiquement dans le mode dilution lorsqu'au moins une condition se produit, dans laquelle la au moins une condition comporte : l'absence de fumée dans le cockpit, le niveau de pression à l'intérieur du cockpit est supérieur à un niveau de pression prédéterminé, la fréquence respiratoire du membre d'équipage est égale à une fréquence respiratoire prédéterminée, le niveau de dioxyde de carbone présent dans le gaz expiré est égal à un niveau de dioxyde de carbone prédéterminé, et la pression partielle d'oxygène présente dans le sang du membre d'équipage est égale à une pression partielle d'oxygène prédéterminée.

7. Le masque respiratoire (102) selon la revendication 1, dans lequel le gaz respiratoire comprend de l'oxygène dans le mode d'urgence et l'air ambiant du cockpit dans le mode de dilution.

8. Le masque respiratoire (102) selon la revendication 1, comportant en outre :
un embout buccal et nasal (202) connecté au régulateur (124) ;
une arrivée de gaz respiratoire (204) et une sortie de gaz respiratoire (206) connectées à l'embout buccal et nasal (202), dans lequel au moins un capteur de fréquence respiratoire (116) de la pluralité de capteurs (104) est disposé dans l'arrivée de gaz respiratoire (204) pour détecter la fréquence respiratoire du membre d'équipage et dans lequel au moins un capteur de dioxyde de carbone (114) de la pluralité de capteurs (104) est disposé dans au moins une sortie de gaz respiratoire (206) et dans l'embout buccal et nasal (202) pour détecter le niveau de dioxyde de carbone présent dans le gaz expiré.

9. Le masque respiratoire (102) selon la revendication 1, comprenant en outre :
un joint facial périphérique (208), dans lequel au moins un capteur (120) de la pluralité de capteurs (104) est disposé autour du joint facial périphérique (208) pour détecter la pression partielle de l'oxygène présent dans le sang du membre d'équipage.

10. Un procédé d'utilisation du masque respiratoire selon la revendication 1, comprenant :
recevoir, d'une pluralité de capteurs (104), des informations associées à de l'air ambiant du cockpit pour au moins un paramètre susceptible d'affecter le niveau d'oxygène et la santé d'un membre de l'équipage pour au moins un paramètre capable de provoquer un trouble respiratoire, et produire des signaux de sortie associés ; et
commuter automatiquement entre les modes de fonctionnement d'un masque respiratoire (102) pour fournir un gaz respiratoire au membre d'équipage sur la base des informations reçues, dans lequel les modes de fonctionnement comportent un mode de dilution, un mode d'urgence et un mode de recirculation, dans lequel le gaz respiratoire comporte une combinaison de gaz expiré et d'oxygène pendant le mode de recirculation.

11. La méthode selon la revendication 10, dans laquelle au moins un paramètre susceptible d'affecter le niveau d'oxygène est choisi dans le groupe constitué par la présence de fumée dans un cockpit, le niveau de pression à l'intérieur du cockpit et les contaminants présents dans l'air ambiant du cockpit.

12. La méthode selon la revendication 10, dans laquelle au moins un paramètre capable de provoquer un trouble respiratoire est choisi dans le groupe constitué de la fréquence respiratoire du membre d'équipage d'un aéronef, du niveau de dioxyde de carbone présent dans les gaz expirés du membre d'équipage, de la pression partielle de l'oxygène présent dans le sang du membre d'équipage, et de la saturation en oxygène des tissus du membre d'équipage.

13. La méthode selon la revendication 10, dans laquelle le gaz respiratoire comprend de l'oxygène dans le mode d'urgence et au moins une combinaison de l'air ambiant du cockpit et de l'air ambiant du cockpit mélangé à de l'oxygène dans le mode de dilution.

14. Méthode de la revendication 10, dans laquelle la commutation automatique entre les modes de fonctionnement pour fournir le gaz respiratoire au membre de l'équipage sur la base des informations reçues, comprend :
commuter automatiquement dans le mode d'urgence lorsqu'au moins une condition d'urgence se produit, dans laquelle la au moins une condition d'urgence comprend la présence de fumée dans le cockpit, le niveau de pression à l'intérieur du cockpit est supérieur à un niveau de pression prédéterminé, la pression partielle de l'oxygène présent dans le sang du membre d'équipage est inférieure à une pression partielle prédéterminée de l'oxygène, le niveau de dioxyde de carbone présent dans le gaz expiré est supérieur à un niveau de dioxyde de carbone prédéterminé, la fréquence respiratoire du membre d'équipage dévie d'une fréquence respiratoire prédéterminée, et la saturation en oxygène des tissus du membre d'équipage est inférieure à une saturation en oxygène des tissus prédéterminée.
